# EUROPEAN PATENT APPLICATION

(11) **EP 3 495 008 A1**
(43) Date of publication of application: **12.06.2019**
(21) Application number: 17306706.7
(22) Date of filing: 06.12.2017
(51) Int. Cl.: A61M 5/20, A61M 5/315, A61M 5/24

(54) **WIND-UP SERVO-ACTUATOR AND DRUG DELIVERY DEVICE**

(71) Applicant: SANOFI, 75008 Paris (FR)
(72) Inventor: HELMER, Michael, 65926 Frankfurt am Main (DE); SCHABBACH, Michael, 65926 Frankfurt am Main (DE); NOBER, Peter, 65428 Rüsselsheim (DE); RAU, Matthias, 65428 Rüsselsheim (DE); KERSTING, Fritz Julian, 65428 Rüsselsheim (DE)
(74) Representative: Sanofi Aventis Deutschland GmbH

(57) **Abstract**

The invention relates to a servo-actuator (10) for driving a push-rod (6), the servo-actuator (10) comprising:
• a housing (11) with a connecting flange (11.6),
• the connecting flange (11.6) being mountable on a proximal section of the housing (2) of the liquid drug delivery device (1);
• a driving spring (18; 118);
• a pusher (16; 130; 240) driveable connected to the driving spring (18; 118),
• the pusher (16; 130; 240) having a mechanical interface (16.6) for engaging with the push-rod (6), when mounted, such that an extension movement of the push-rod (6) results in energizing of the compression spring (18; 118) and a subsequent returning movement of the push-rod (6) is propelled by energy supplied from the compression spring (18; 118),
• the mechanical interface (16.6) further being configured for selectively interacting with a trigger (7) located at a terminal portion of the piston-rod (4) of the liquid drug delivery device (1), when mounted; and
• a controllable latch (21) for allowing a user to selectively prevent and release the pusher (16; 130; 240) from being driven by the force of the compression spring (18), when energized,
• whereby the controllable latch (21) is connected to the mechanical interface (16.6) of the pusher (16; 130; 240) such that the mechanical interface (16.6) is selected to interact with the trigger (7) when the controllable latch (21) is released.

## Description

### Technical Field

The disclosure generally relates to a drug delivery device for an injection, wherein a dosage of a medicament can be chosen or dialled before the injection. More particularly, the disclosure generally relates to a servo-actuator that is adapted to be mounted onto such a drug delivery device and to be wind up when a dosage is dialled before the injection. Still more particularly, the disclosure relates to a servo-actuator providing a button and a spring storage that is wind up or loaded when the button is in a dialling position and wherein the spring storage is unloaded when the button is in an injection position, thereby driving the drug delivery device such that the dialled dosage is delivered.

The disclosure further relates to a system comprising such a drug delivery device and such a servo-actuator.

### Background

Insulin and other injectable medications are commonly given with drug delivery pens, whereby a disposable pen needle assembly is attached to facilitate drug container access and allow fluid egress from the container through the needle into the patient.

Advancement in technology and competition drives the desire for shorter, thinner, less painful, and more efficacious injections, the design of the pen needle assembly and parts thereof becomes more and more important. In particular, device designs may need to proactively address ergonomically improving injection technique, injection depth control and accuracy, the ability to be safely used and transported to disposal, and protection against misuse while maintaining the ability to be economically manufactured on a mass production scale.

Existing intradermal drug delivery devices may, in circumstances, require increased operational force to inject the medication because of back pressure created from the intradermal layer, thereby making the intradermal injection difficult. Accordingly, a need may become apparent in certain situation for facilitating the manual operation of a drug delivery pen.

### Summary

According to the disclosure a servo-actuator for driving a push-rod, in particular a helically extending and returning push-rod of a drug delivery device is provided. The servo-actuator comprises at least a housing with a connecting flange, wherein the connecting flange being mountable on a proximal section of the housing of the liquid drug delivery device. The servo-actuator further comprises a driving spring and a pusher driveable connected to the driving spring. The pusher has a mechanical interface for engaging with the push-rod, when mounted, such that an extension movement of the push-rod results in energizing of the compression spring and a subsequent returning movement of the push-rod is propelled by energy supplied from the compression spring. The mechanical interface is further being configured for selectively interacting with a trigger located at a terminal portion of the piston-rod of the liquid drug delivery device, when mounted. The servo-actuator further comprises a controllable latch for allowing a user to selectively prevent and release the pusher from being driven by the force of the compression spring, when energized, whereby the controllable latch is connected to the mechanical interface of the pusher such that the mechanical interface is selected to interact with the trigger when the controllable latch is released.

In an exemplary embodiment, the driving spring is configured as a linear spring or compression spring, wherein a proximal end of the driving spring is coupled to the housing and a distal end of the driving spring is coupled to the pusher.

In a further exemplary embodiment, the driving spring is a pre-loaded helical spring or a preloaded spiral spring, wherein a proximal end of the driving spring is coupled to the housing and a distal end of the driving spring is coupled to the pusher.

The servo-actuator may further comprise a dial grip adapted to transmit a torque to the push-rod.

For example, the pusher may be configured for coupling with the push-rod of the drug delivery device and for coupling with the housing. In particular, the pusher comprises a receptacle for receiving and holding a proximal end of the push-rod.

In another exemplary embodiment, the pusher comprises a lead for relative threaded engagement to the housing, in particular indirectly via the thread of the guide sleeve.

In an exemplary embodiment, the lead of the pusher relative to the housing equals a lead of the thread which guides the push-rod relative to the device housing.

Furthermore, the servo-actuator may comprise a button axially movable relative to the housing between a proximal dialling position and a distal injection position, wherein the pusher is engaged relative to the housing when the button is in its proximal dialling position and wherein the pusher is disengaged relative to the housing when the button is in its distal injection position.

In an exemplary embodiment, the controllable latch is configured as an engagement force applied by friction of lead connections or a force fit connection between the pusher and a support sleeve, in particular a latching sleeve or a guide sleeve.

Further, the pusher may be formed as a rotatable pusher coupled with the button and in threaded engagement with a non-rotatable guide sleeve, in particular the guide sleeve is urged proximally against the pusher by a support spring, thereby frictionally engaging the pusher relative to the guide sleeve such that the unloading of the driving spring is frictionally inhibited when the button is in its proximal dialling position and frictionally disengaging the pusher relative to the guide sleeve when the button absorbs the spring force of the support spring in its distal injection position.

Furthermore, the button may be rotationally coupled with the pusher and rotationally interlaced with the guide sleeve, wherein the button is moveable from its proximal dialling position into its distal injection position only at discrete angular positions of the pusher relative to the guide sleeve.

According to the disclosure, the pusher is received in a radially resilient interior latching sleeve that is axially slidably received in an exterior latching sleeve and axially coupled with the button, in particular the interior latching sleeve is urged proximally against the exterior latching sleeve and against the button by a support spring and further in particular the latching sleeves are radially interlaced such that the inner circumference of the interior latching sleeve tightens in a proximal position, thereby wedging the pusher relative to the interior latching sleeve, and loosens in a distal position, thereby releasing the pusher relative to the interior latching sleeve, such that the unloading of the driving spring is inhibited when the button is in its proximal dialling position and released when the button is in its distal injection position.

The invention relates further to a drug delivery device with a device housing extending along a longitudinal axis and receiving a cartridge, a push-rod with a trigger protruding from the distal end of the device housing, a piston rod adapted to drive a piston of the cartridge with a liquid drug, and a servo-actuator as described above and below further and which is being removably mounted on the device housing.

In an exemplary embodiment, the servo-actuator is configured as an add-on which is removably coupled to a proximal end of the device housing.

Further scope of applicability of the present disclosure will become apparent from the detailed description given hereinafter. However, it should be understood that the detailed description and specific examples, while indicating exemplary embodiments of the disclosure, are given by way of illustration only, since various changes and modifications within the spirit and scope of the disclosure will become apparent to those skilled in the art from this detailed description.

### Brief Description of the Drawings

The present disclosure will become more fully understood from the detailed description given below and the accompanying drawings, which are given by way of illustration only, and do not limit the present disclosure, and wherein:
- Figure 1: is a schematic perspective view on a first embodiment of a servo-actuator mounted on the proximal end of an drug delivery device,
- Figures 2 and 4: are schematic longitudinal sections of a first embodiment of a servo-actuator,
- Figure 3: is a schematic perspective view on a support sleeve according to a first embodiment of a servo-actuator,
- Figures 4A, 5B, 6A and 6B: are schematic sections of a second embodiment of a servo-actuator in a dialling position,
- Figure 7: is a schematic section of a second embodiment of a servo-actuator in an injection position,
- Figure 8: is a schematic section of a third embodiment of a power pack-add on in a dialling position
- Figure 9: is a schematic section of a wedge sleeve and of an auxiliary sleeve and
- Figures 10A and 10B: are schematic sections of a third embodiment of a servo-actuator in an injection position.

Corresponding parts are marked with the same reference symbols in all figures.

### Detailed Description

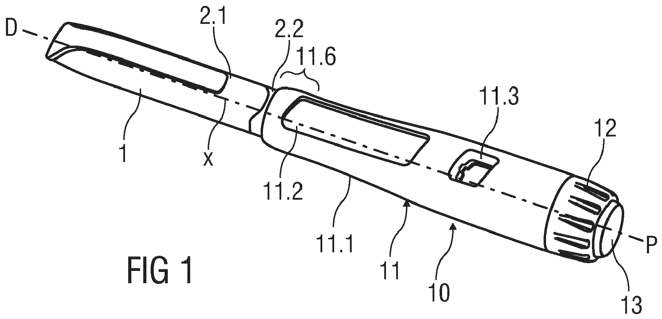

**Figure 1** shows a perspective view on a drug delivery device 1 having a servo-actuator 10 according to the present disclosure mounted on its proximal end P.

The drug delivery device 1 implements the mechanical scheme of the dial-extension mechanism as, for example, the SoloSTAR® injection pen which is in more detail described in the published applications WO2004/078239 A1, WO2004/078240 A1 and WO2004/078241 A1. Alternatively, another marketed implementation of a dial-extension mechanism may be taken from Novo Nordisk's FlexPen® pen injector as described in WO 99/38554 A1.

In an exemplary embodiment, the drug delivery device 1 comprises a device housing 2.

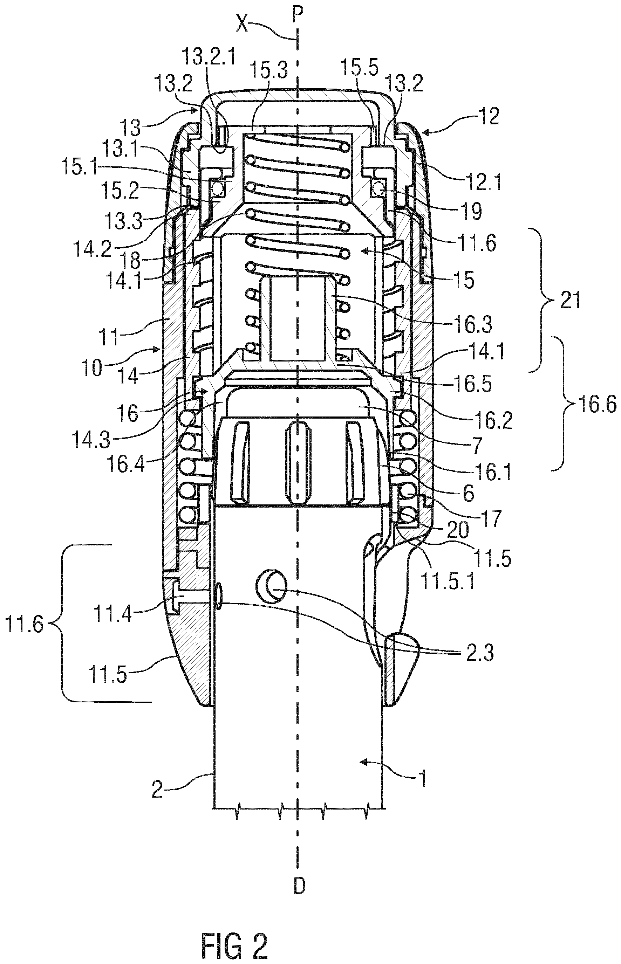

According to the disclosure, the device housing 2 may be configured as a one-piece housing. In figure 2, the device housing 2 comprises two housing parts 2.1, 2.2. The device housing 2 has substantially a cylindrical shape. In particular, the device housing 2 has a substantially constant diameter along the longitudinal axis X.

The drug delivery device 1 has a distal end D and a proximal end P. The term "distal" refers to a location that is relatively closer to a site of injection, and the term "proximal" refers to a location that is relatively further away from the injection site.

In an exemplary embodiment, the servo-actuator 10 comprises an essentially tubular housing 11 that covers a proximal section of the device housing 2, e.g. housing part 2.2, and the push-rod 6 with the trigger 7.

The housing 11 may extend in the distal direction and provide an ergonomically formed grip area 11.1 for improved handling of the drug delivery device 1. The housing 11 may further provide a recess or label window 11.2 for arranging a label and/or a recess or dosage indicator window 11.3 arranged for observation of a dosage indicator of the drug delivery device 1. The housing 11 further comprises the connecting flange 11.6 at its distal end.

On its proximal end, the servo-actuator 10 provides a dial grip 12 that is rotatable relative to the housing 11. On the end surface of the dial grip 12, a button 13 is arranged. The button 13 is axially movable along the longitudinal axis between a proximal or dialling position and a distal or triggering position.

The servo-actuator 10 is for example configured as an add-on, an adaptor, an additional assembly, a power pack or a support element for the drug delivery device 1 configured as a self-injector.

The servo-actuator 10 is further configured as a medical wind-up element providing automatic self-injection.

The servo-actuator 10 or add-on comprises at least one coupling sized and shaped to couple the servo-actuator 10 to the device housing 2 of the drug delivery device 1, e.g. to a proximal end P of the device 1.

A coupling is formed as the connecting flange 11.6. The connecting flange 11.6 is being mountable on a proximal section of the housing 2 of the drug delivery device 1.

The servo-actuator 10 coupled to the drug delivery device 1 forms an integral self-injector system. The servo-actuator 10 can be removably or reusably coupled to the drug delivery device 1.

The servo-actuator 10 serves as a support for injection. In particular, the servo-actuator 10 is configured to cover the moving out of a number sleeve (not shown) of the drug delivery device 1. Further, the servo-actuator 10 is such configured and coupled to the drug delivery device 1 that an automatic injection can be carried out even though the drug delivery device 1 is a manual device.

**Figure** 2 shows the servo-actuator 10 in more detail and in an assembled state onto the proximal end P of the drug delivery device 1.

The servo-actuator 10 further comprises a driving spring 18 and a pusher 16 which is driveable connected to the driving spring 18. The pusher 16 has a mechanical interface 16.6 for engaging with the push-rod 6 of the drug delivery device 1, when mounted, such that an extension movement of the push-rod 6 results in energizing of the driving spring 18 and a subsequent returning movement of the push-rod 6 is propelled by energy supplied from the driving spring 18. In an exemplary embodiment, the mechanical interface 16.6 comprises a receptacle 16.4 for receiving the proximal end P of the drug delivery device 1.

The mechanical interface 16.6 is further being configured for selectively interacting with a trigger 7 located at the proximal end P, e.g. at a terminal portion of a piston-rod (not shown) of the liquid drug delivery device 1, when mounted.

The servo-actuator 10 further comprises a controllable latch 21 for allowing a user to selectively prevent and release the pusher 16 from being driven by the force of the driving spring 18, when energized, whereby the controllable latch 21 is connected to the mechanical interface 16.6 of the pusher 16 such that the mechanical interface 16.6 is selected to interact with the trigger 7 when the controllable latch 21 is released.

In an exemplary embodiment, the controllable latch 21 is configured as an engagement force applied by friction of lead connections, thread connections or other force fit connections between the pusher 16 and a guide sleeve 14, e.g. a threaded sleeve.

**Figure 2** shows a longitudinal section of a first embodiment of a servo-actuator 10. The servo-actuator 10 is fixated relative to the drug delivery device 1 in the area of the connecting flange 11.6 e.g. by means of radial fixing screws 11.4 that radially reach through a socket 11.5 arranged at the distal end of the housing 11. The socket 11.5 protrudes radially inwardly. The fixing screws 11.4 are received in corresponding radial blind fixing holes 2.3 of the device housing 2. In an alternative embodiment, the housing 11 of the servo-actuator 10 can be adhesively fixated at the device housing 2, for example by plastic welding or by an adhesive.

Inside its housing 11, the servo-actuator 10 comprises a cylindrical guide sleeve 14, a support sleeve 15, a pusher leg or pusher rod, a support spring 17 and a driving spring 18.

The dial grip 12 is formed for example as a sleeve and arranged at the proximal end of the servo-actuator 10. The dial grip 12 receives the button 13 which is formed as a tubular case with a proximal front face. On its outer tubular surface, the button 13 provides protrusions 13.1 that engage corresponding pockets12.1 in the inner surface of the dial grip 12, thereby rotationally coupling the button 13 with the dial grip 12. The pockets 12.1 stretch coaxially to the longitudinal axis X, thereby providing an axial play for the button 13 relative to the dial grip 12 between a most proximal or dialling position and a most distal or injection position. On its inner tubular surface, the button 13 provides recesses 13.2.1 along an inner rim 13.2. A distal rim 13.3 of the tubular case forming the button 13 abuts a proximal rim 14.2 of the guide sleeve 14.

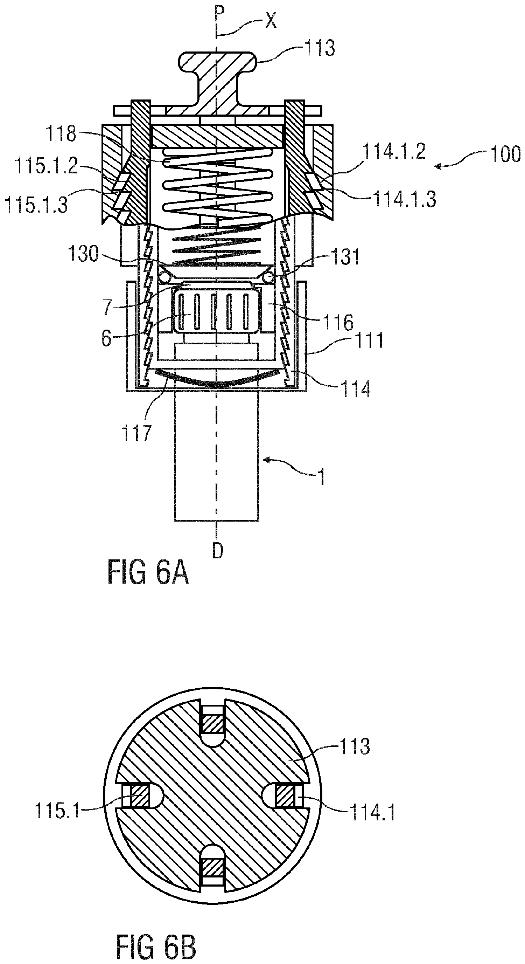

The guide sleeve 14 is rotationally coupled with the housing 11, but axially movable along the longitudinal axis X relative to the housing 11. Threads 14.1 or pitches are arranged on the inner surface of the guide sleeve 14. A distal rim 14.3 of the guide sleeve 14 abuts the proximal end of the support spring 17 which is arranged between the socket 11.5 and the guide sleeve 14, thereby enclosing the push-rod 6 and the trigger 7 of the drug delivery device 1. Thereby, the support spring 17 urges, via the guide sleeve 14, the button 13 proximally towards its proximal or dialling position. The support spring 17 is formed as a coil spring. Alternatively, the support spring 17 may be formed differently, for example as a plate spring 117 (shown in figures 6A, 7A, 8, 9), as long as it urges the button 13 proximally and allows a sufficient longitudinal axial displacement of the button 13 and the guide sleeve 14.

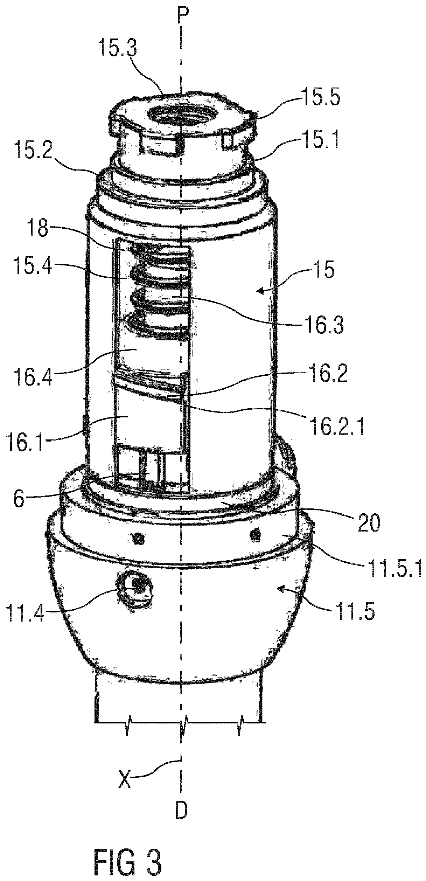

**Figure 2** shows the proximal end of the drug delivery device 1 with the coupled servo-actuator 10. **Figure 3** shows the support sleeve 15 in more detail.

The guide sleeve 14 receives the support sleeve 15, wherein a tapering dome 15.1 of the support sleeve 15 proximally protrudes the guide sleeve 14 and is received within the button 13. The dome 15.1 provides a radial step 15.2 or tapering and a proximal rim 14.2. The radial step 15.2 abuts a supporting collar 11.7 of the housing 11, wherein a first ring 19 is optionally arranged between the radial step 15.2 and the support collar 11.7 to reduce axial and radial friction.

The distal end of the support sleeve 15 is supported by the socket 11.5. A radial bearing formed as a second ring 20 is optionally arranged enclosing a socket collar 11.5.1 and the distal end of the support sleeve 15.

The rings 19, 20 are made of a friction-reducing material in order to ease a rotation of the support sleeve relative to the housing 11. In an embodiment, the rings 19, 20 are made of polyoxymethylene. Depending on used material of the housing 11 and the support sleeve 15 optional rings 19, 20 may be omitted.

On the rim 15.3, radial protrusions 15.5 are formed that engage corresponding recesses 13.2.1 along the inner rim 13.2 in the inner surface of the button 13, when the button 13 is in its proximal or dialling position, thereby rotationally coupling the support sleeve 15 with the button 13. When the button 13 translates distally relative to the support sleeve 15, the radial protrusions 15.5 disengage from the recesses 13.2.1, thereby rotationally uncoupling from the support sleeve 15.

The support sleeve 15 receives the pusher 16. The pusher 16 is formed for example as a pusher such that the pusher 16 is rotationally coupled but axially translatable along the longitudinal axis X in relation to the support sleeve 15.

As shown in more detail in **figure 3****,** axial slots 15.4 extend along the longitudinal axis X from the dome 15.1 towards the distal end of the support sleeve 15. The axial slots 15.4 longitudinally guide slides 16.1 which protrude radially outwardly from the pusher 16.

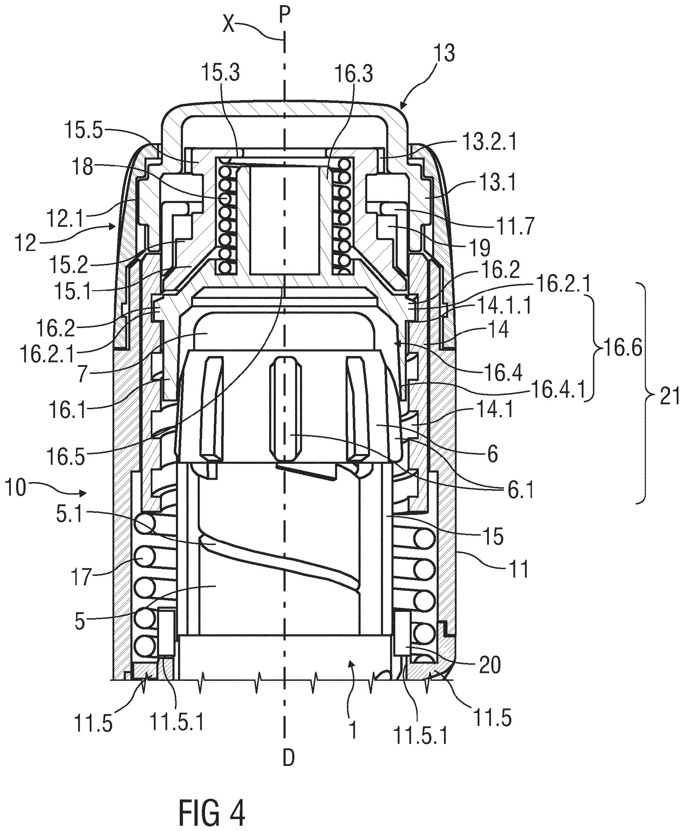

On the outer surface of each slide 16.1, a partial helical lead 16.2 is formed. As can be seen again from figure 2 and 4, those leads 16.2 are guided in the helical threads 14.1 or pitches of the guide sleeve 14. Along the longitudinal axis X, the width of the leads 16.2 is sufficiently smaller than the width of the threads 14.1 in order to provide a longitudinal axial play between the guide sleeve 14 and the pusher 16.

The flank lead of the threads 14.1 on the inner surface of the guide sleeve 14 is chosen equivalent to the flank lead of the threads that guide the push-rod 6 relative to the device housing 2. In other words: the threads 14.1 are chosen such that the upon a complete turn relative to the guide sleeve 14, the pusher 16 travels along the same distance in the same direction along the longitudinal axis X, as the push-rod 6 travels upon a complete turn in relation to the device housing 2.

Inside the support sleeve 15, the driving spring 18 is arranged between the rim 15.3 and the pusher 16. In order to improve the support of the driving spring 18, a proximally facing spring bearing 16.3 may be arranged at the pusher 16. As the support sleeve 15 is longitudinally supported by the socket 11.5 and by the collar 11.5.1, the pusher 16 is urged distally along the longitudinal axis X in relation to the housing 11.

**Figure 4** shows a schematic longitudinal section of the servo-actuator 10 with the button 13 in its proximal dialling position.

When the button 13 is in its proximal dialling position, the support spring 17 urges the guide sleeve 14 relative to the housing 11 into a proximal direction, such that a proximal face 14.1.1 of the thread 14.1 frictionally abuts a distal face 16.2.1 of a lead 16.2 led therein. The friction at the interface between the proximal face 14.1.1 and the distal face 16.2.1 is determined by the resulting force of the support spring 17 and the driving spring 18 that apply pressure in opposite directions on said interface. Both springs 17, 18 are pre-compressed when assembled in the servo-actuator 10. The springs 17, 18 are chosen such that the restoring force of the support spring 17 exceeds the restoring force of the driving spring 18 even if the driving spring 18 is maximally deflected, i.e. in its pre-compressed status upon assembly. Furthermore, the driving spring 18 is chosen such that it overcomes the trigger restoring force in its pre-compressed status.

On its distal end, the pusher 16 provides a receptacle 16.4 that is adapted to receive the push-rod 6. On its inner surface, the receptacle 16.4 provides longitudinal notches 16.4.1 that are adapted to receive the peripheral ridges 6.1 of the push-rod 6, thereby rotationally coupling the pusher 16 to the push-rod 6. On its proximal end, the receptacle 16.4 is closed by a lid 16.5 supporting the spring bearing 16.3.

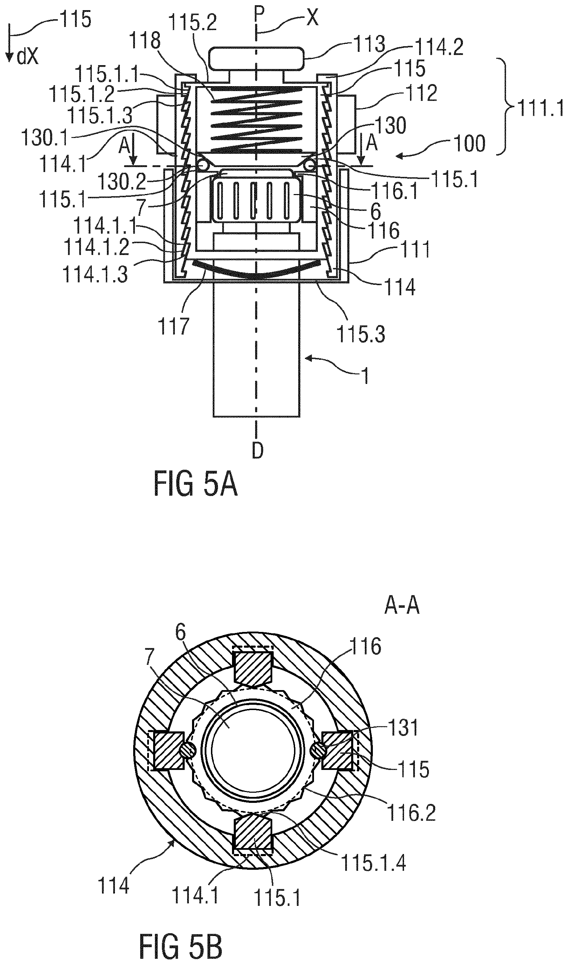

The axial position of the pusher 16 relative to the guide sleeve 14 can be adjusted before assembly of both the servo-actuator 10 and the drug delivery device 1 by rotation of the pusher 16 relative to the guide sleeve 14 into a mounting position. The axial position is chosen such that in this mounting position with the button 13 in its most proximal dialling position, the lid 16.5 of the receptacle 16.4 is proximally displaced from the trigger 7, while this axial displacement dX (shown in figure 5) is within the axial play of the button 13.

In the following, the dialling of the drug delivery device 1 is explained in more detail.

For dialling, the button 13 is in its most proximal dialling position and is rotationally coupled with the rim 15.3 of the support sleeve 15. When a torque is exerted upon the dial grip 12, the torque is transferred via the button 13, the support sleeve 15, the pusher 16 and the receptacle 16.4 positively fitting the push-rod 6. Due to the same flank lead of their threaded guidance, both the push-rod 6 and the pusher 16 travel synchronously along the longitudinal axis X, such that the distance between the trigger 7 and the receptacle lid 16.5 is maintained. The support sleeve 15 follows the rotation of the pusher 16, as the slides 16.1 engage the axial slots 15.4, but maintains its axial position relative to the housing 11.

Depending on the axial position of the pusher 16, the dial grip 12 may be turned in a right-handed and/or in a left-handed direction. A right-handed turning of the dial grip 12 up-dials the dosage and charges the driving spring 18. A left-handed turning of the dial grip 12 down-dials the dosage and discharges the driving spring 18.

When the pusher 16 travels proximally, it charges the driving spring 18.

**Figure 4** schematically shows the situation with a fully charged driving spring 18 after dialling a maximum dosage.

The push-rod 6 may drive a number sleeve 5 of the drug delivery device 1 such that the dialed or chosen dosage is visually indicated, as known from the state of the art. Preferably, a dosage indicator window 11.3 (shown in figure 2) is arranged in the housing 11 such that this visual indication is visible to the user.

The friction between the proximal face 14.1.1 of the guide sleeve 14 and the distal face 16.2.1 of the pusher 16 maintains the rotational position and thus the axial position of the pusher 16. When the user presses the button 13 in a distal direction and overcomes the restoring force of the support spring 17, both the button 13 and the guide sleeve 14 travel distally. Due to its threaded engagement, the pusher 16 follows the axial travel of the support sleeve 15 until the distal face of the receptacle lid 16.5 abuts the proximal face of the trigger 7.

The trigger 7 is impacted by a trigger restoring force of a biasing means that urges the trigger 7 in a proximal direction. Upon further movement of the button 13 in a distal direction, the trigger restoring force counteracts the spring force that the driving spring 18 exerts upon the receptacle 16.4 in a distal direction. Thus upon further distal movement of the button 13, the axial force pressing the distal face 16.2.1 of the lead 16.2 towards the proximal face 14.1.1 of the threads 14.1 is reduced by the trigger restoring force, while the trigger 7 is pressed distally towards its injection position.

The threading angle and / or the surface coarseness of the thread 14.1 and the lead 16.2 are chosen in such a way that the static friction caused by the unreduced spring force of the driving spring 18 reliably locks the pusher 16 relative to the guide sleeve 14 and, thus, the controllable latch 21, whereas the driving spring force is insufficient to lock the pusher 16 when counteracted by the trigger restoring force. As an example, either one or both of the faces 14.1.1, 16.2.1 may provide engaging ridges, burlings, bumps or teeth in order to increase and better control the friction between the lead 16.2 and the thread 14.1

When the button 13 is pressed further distally towards its most distal injection position, the receptacle lid 16.5 abuts and then triggers the trigger 7, thereby unlocking the drug delivery device 1, e.g. the controllable latch 21 releases guide sleeve 14 from the pusher 16. In particular, the button 13 is pressed inwards so that a proximal end of the guide sleeve 14, in particular the proximal face 14.1.1, is being bent slightly outward so that the pusher 16 comes free to move distally. The charged driving spring 18 can thus discharge by driving the pusher 16 in a distal direction. The receptacle 16.4 drives the trigger 7 and the device dialling grip 6 in a distal direction, thereby administering the dialled dosage of the liquid drug.

As the pusher 16 and the push-rod 6 travel distally, they turn synchronously due to their equal flank lead. The discharging of the driving spring 18 is limited by the most distal axial position of the push-rod 6 that corresponds to a zero dosage. However, the discharging of the driving spring 18 and thus the administration of the drug may be paused when the user releases the button 13. Then the support spring 17 urges the guide sleeve 14 with the engaged pusher 16 in a distal direction such that the receptacle lid 16.5 no longer abuts the trigger 7. Thus, the static friction is no longer reduced by the trigger restoring force. The administering of the drug can be resumed by pressing the button 13. If the user releases the button 13 the proximal face 14.1.1 of the guide sleeve 14 flexes back, in particular inwardly and the guide sleeve 14 and the pusher 16 come into contact with each other again so that distal movement of the pusher 16 is stopped. Pausing and resuming can be repeated until the pre-dialled dosage is completely administered and the push-rod 6 is back in its zero dosage position.

It is an advantage of this embodiment that a chosen dosage is administered by mere triggering of the button 13, such that the user is relieved of translationally urging a handle that is axially coupled to the piston rod 4. Thereby, a safer, smoother and more convenient application of the injection is reached.

It is a further advantage of this embodiment that the mechanical size, specifically the overall length of the assembly of the drug delivery device 1 and the servo-actuator 10 is constant and independent of the dialled dosage. Thus, a drug delivery device 1 with a pre-dialled dosage may be stored in the same way as after the administration of a drug, thereby reducing the risk of an inadvertent triggering of a pre-loaded drug delivery device 1.

In an embodiment, the button 13 is form-fittingly coupled to the housing 11 such that its axial movement is enabled in certain discrete angular positions, while it is axially locked in between these angular positions. Thereby, only discrete dosages of the liquid medicament can be applied. For example, a pin-slot coupling could prevent triggering of the button 13 in all but one angular position. Then, only a dosage that is a multiple of the dosage corresponding to a complete turn of the push-rod 6 can be applied. It is an advantage of this embodiment that the safety and reliability of the application of a drug is improved.

**Figure 5A** shows a second embodiment of a servo-actuator 100 mounted on a proximal end of a drug delivery device 1 according to the state of the art. The servo-actuator 100 comprises a housing 111 with a grip area 111.1 and a dial grip 112 which is rotatable relative to the housing 111. The servo-actuator 100 further comprises a button 113.

The housing 111 encloses an exterior latching sleeve 114. An interior latching sleeve 115 is led inside the exterior latching sleeve 114 and rotationally coupled thereto. As can be seen from **Figure 6B****,** the exterior latching sleeve 114 has a substantially circular cross section. On its inner surface, longitudinal guiding slots 114.1 are arranged that stretch along the longitudinal axis X. The guiding slots 114.1 receive resilient latch arms 115.1 connecting a proximal lid 115.2 with a distal bottom 115.3, thereby forming a cage-like interior latching sleeve 115.

The radially inward face of the slots 114.1 and the radially outward face of the latch arms 115.1 provide correspondingly inclined slot teeth 114.1.1 and arm teeth 115.1.1, respectively.

Upon a distal displacement dX of the interior latching sleeve 115 relative to the exterior latching sleeve 114, the inclined teeth faces 114.1.2, 115.1.2 slide along each other. In a distal position of the interior latching sleeve 115, the proximal front faces 114.1.3 of the slot teeth 114.1.1 abut the distal front faces 115.1.3 of the arm teeth 115.1.1. In a proximal position of the interior latching sleeve 115, the lid 115.2 abuts a stop 114.2 that radially inwardly protrudes on the proximal end of the exterior latching sleeve 114. The interior latching sleeve 115 is urged towards its proximal position by a spring plate 117 arranged between the housing 111 and the distal bottom 115.3.

Inside the interior latching sleeve 115 an auxiliary sleeve 116 is arranged that positively fittingly receives the push-rod 6, such that it is rotationally coupled thereto, while it is translationally movable along the longitudinal axis X relative to the interior latching sleeve 115. On its proximal end, a radially inwardly protruding stop 116.1 limits a distal translation of the auxiliary sleeve 116 relative to the push-rod 6 such that the trigger 7 proximally protrudes the proximal end of the auxiliary sleeve 116. The auxiliary sleeve 116 is furthermore rotationally coupled to the interior latching sleeve 115, as the radially inwardly facing profile of the latch arms 115.1 provides a ridge 115.1.4 that engages a correspondingly formed chamfer 116.2 in the outward perimeter of the auxiliary sleeve 116, as can be seen from **Figure 5B****.**

Inside the proximal end of the interior latching sleeve 115, a driving spring 118 and a pushing plate 130 are arranged. On its distal face, the pushing plate 130 provides a distally tapering inclined rim 130.1. The distal end of the driving spring 118 urges the pushing plate 130 in a distal direction towards the auxiliary sleeve 116 enclosing the push-rod 6. The proximal end of the driving spring 118 can be borne by the lid 115.2, as shown in **Figure 5A****.**

Between the proximal face of the stop 116.1 and the distal face of the rim 130.1, balls 130.2 are arranged. The diameter of the balls 130.2 is chosen such that the distal face of the pushing plate 130 is offset from the proximal face of the trigger 7, while the pushing plate 130 rests via the balls 130.2 on the proximal face of the auxiliary sleeve 116. By means of the stop 116.1, the auxiliary sleeve 116 is prevented from giving way to the pressure applied by the pushing plate 130, as long as the trigger 7 in its proximal dialling position locking the drug delivery device 1.

A dosage is dialled by turning the dial grip 112 with the button 113 in its dialling position. The torque is transmitted from the dial grip 112 to the exterior latching sleeve 114 and further on to the interior latching sleeve 115 and the auxiliary sleeve 116. The auxiliary sleeve 116 transfers the torque onto the push-rod 6. The threaded engagement of the push-rod 6 translates a rotation into a translation along the longitudinal axis X, wherein the driving spring 118 is loaded upon up-dialling or unloaded upon down-dialling, until the desired dosage is set.

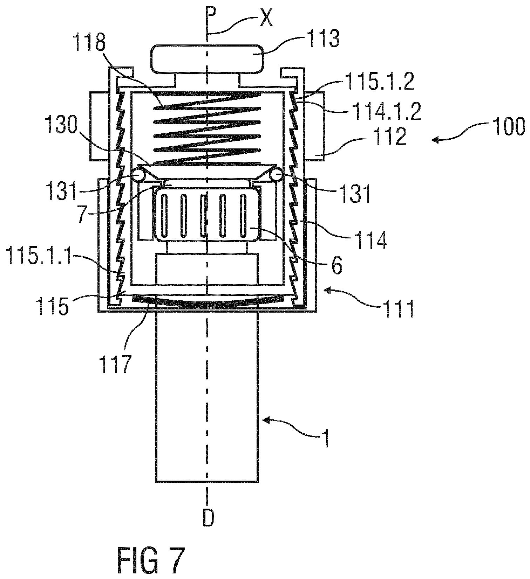

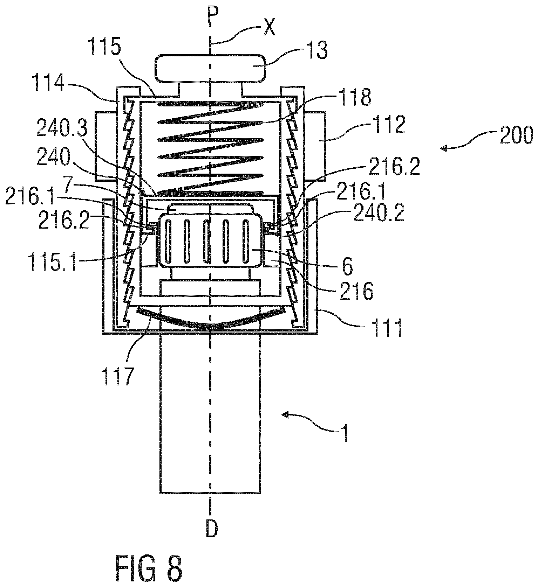

When the user presses the button 113, thereby overcoming the restoring force of the spring plate 117, the interior latching sleeve 115 is translated distally towards its distal end position. The resilient latch arms 115.1 flex radially outwardly as they are urged by the balls 130.2 being under pressure from the pushing plate 130, as can be seen in **figure 8****.** When the balls 130.2 move radially outwardly, shown in **figure 7****,** the pushing plate 130 approaches, abuts and then triggers the trigger 7, thereby unlocking the drug delivery device 1 such that the dialled dosage is administered.

When the user releases the button 113, the interior latching sleeve 115 is urged proximally by the spring plate 117. The inclined faces 115.1.2 of the interior latching sleeve 115 slide along the inclined faces 114.1.2 of the exterior latching sleeve 114, thus tightening the interior latching sleeve 115. The balls 130.2 are urged radially inwardly, thereby setting the pushing plate 130 off the trigger 7. The drug delivery device 1 is thus locked again.

As an advantage, this embodiment is particularly easy to manufacture. It is furthermore particularly versatile as it is independent of the threading of the push-rod 6 relative to the device housing 2.

**Figure 8** shows a longitudinal section of a third embodiment of a servo-actuator 200. This third embodiment differs from the second embodiment in that a longitudinal translation of the driving spring 118 is inhibited by a wedge sleeve 240 instead of balls 130.2. The wedge sleeve 240 is rotationally paired with the latch arms 115.1 of the interior latching sleeve 115. As a further difference, a modified auxiliary sleeve 216 provides a rim 216.1 on its proximal end.

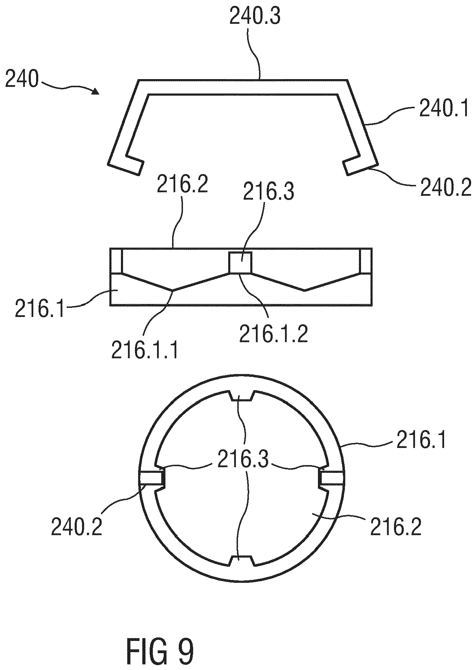

As can be seen better by **figure 9****,** the wedge sleeve 240 provides outwardly curved resilient arms 240.1 with inwardly protruding latches 240.2. The arms 240.1 are arranged on both ends of a beam 240.3. The resilient arms 240.1 abut the latch arms 115.1. The longitudinal profile of the rim 216.1 varies along its perimeter such that the rim 216.1 provides valleys 216.1.1 and hills 216.1.2. A proximally protruding collar 216.2 is arranged radially inwardly of the rim 216.1. The collar 216.2 provides longitudinal slots 216.3 that are adapted to receive the latches 240.2. The angular position of the slots 216.3 coincides with the top of a hill 216.1.2 along the profile of the rim 216.1.

When the button 113 and the interior latching sleeve 115 are in their proximal positions, the latch arms 115.1 of the interior latching sleeve 115 are narrowed as explained with the second embodiment. Thereby, the resilient arms 240.1 of the wedge sleeve 240 are tightened, wherein the latches 240.2 are forced into the slots 216.3, such that the wedge sleeve 240 is rotationally coupled with the auxiliary sleeve 216.

A torque applied on the dial grip 112 is transferred via the exterior latching sleeve 114 and the interior latching sleeve 115 onto the wedge sleeve 240 and further onto the auxiliary sleeve 216 that rotates the push-rod 6. Upon rotation, the push-rod 6 translates longitudinally and drives the wedge sleeve 240 along the longitudinal axis X. Thereby, the driving spring 118 is loaded when the push-rod 6 is up-dialed or unloaded when the push-rod 6 is down-loaded, until the desired dosage is set.

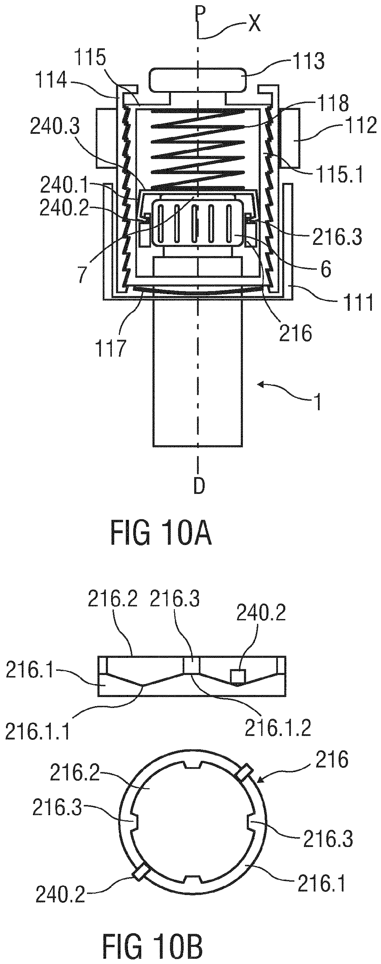

When the user presses the button 113, thereby overcoming the restoring force of the spring plate 117, the interior latching sleeve 115 is translated distally towards its distal end position. The resilient latch arms 115.1 flex radially outwardly as they are urged by the resilient wedge arms 240.1, as can be seen in **figure 10A** and **10B****.** Further, the pusher or latching sleeve 115 is rotationally locked to the support or wedge sleeve 240, e.g. by ridges, guides, slots or teeth. As the interior latching sleeve 115 is moved distally the rotational locking fit between wedge sleeve 240 and interior latching sleeve 115 is being unlocked so that the dial grip 112 does not rotate while pressing the button 113.

As the wedge arms 240.1 bend outwardly, the latches 240.2 detach from the locked position in the slots 216.3 such that the wedge sleeve 240 is rotationally unlocked from the auxiliary sleeve 216. As urged by the driving spring 118, the latches 240.2 travel distally along the perimeter of the rim 216.1 into the nearest valley 216.1.1, thereby causing a slight rotation and a distal displacement of the wedge sleeve 240 in relation to the auxiliary sleeve 216, as can be seen in figures 10A and 10B. The distal displacement causes the beam 240.3 to abut and then to trigger the trigger 7, such that the drug delivery device 1 is unlocked. The loaded driving spring 118 unloads and drives the trigger 7 with the device dial sleeve 6 distally such that the dialed dosage is administered.

When the user releases the button 113, the interior latching sleeve 115 is urged proximally by the spring plate 117. As the latches 240.2 still reside in the valleys 216.1.1, the wedge sleeve 240 wedges the interior latching sleeve 115 and prevents it from travelling in a proximal direction. When the user slightly rotates the dial grip 112 to initiate a subsequent dialing, the wedge sleeve 240 is driven such that the latches 240.2 align with the slots 216.3. Then, the latches 240.2 are urged into the slots 216.3 by the pressure force of the spring plate 117 that is redirected into a radial inwardly direction by the inclined faces 114.1.2, 115.1.2. Thereby, the auxiliary sleeve 216 is rotationally locked with the wedge sleeve 240 again and the wedge sleeve 240 gives way to a proximal displacement of the interior latching sleeve 115 such that the button 13 returns into its distal dialing position.

It is an advantage of this embodiment that is particularly easy to manufacture. It is furthermore particularly versatile as it is independent of the threading of the push-rod 6 relative to the device housing 2.

The drug delivery device, as described herein, may be configured to inject a drug or medicament into a patient. For example, delivery could be sub-cutaneous, intra-muscular, or intravenous. Such a device could be operated by a patient or care-giver, such as a nurse or physician, and can include various types of safety syringe, pen-injector, or auto-injector.

The device can include a cartridge-based system that requires piercing a sealed ampule before use. Volumes of medicament delivered with these various devices can range from about 0.5 ml to about 2 ml. Yet another device can include a large volume device ("LVD") or patch pump, configured to adhere to a patient's skin for a period of time (e.g., about 5, 15, 30, 60, or 120 minutes) to deliver a "large" volume of medicament (typically about 2 ml to about 5 ml).

In combination with a specific medicament, the presently described devices may also be customized in order to operate within required specifications. For example, the device may be customized to inject a medicament within a certain time period (e.g., about 3 to about 20 seconds for auto-injectors, and about 10 minutes to about 60 minutes for an LVD). Other specifications can include a low or minimal level of discomfort, or to certain conditions related to human factors, shelf-life, expiry, biocompatibility, environmental considerations, etc. Such variations can arise due to various factors, such as, for example, a drug ranging in viscosity from about 3 cP to about 50 cP. Consequently, a drug delivery device will often include a hollow needle ranging from about 25 to about 31 Gauge in size. Common sizes are 27 and 29 Gauge.

The delivery devices described herein can also include one or more automated functions. For example, one or more of needle insertion, medicament injection, and needle retraction can be automated. Energy for one or more automation steps can be provided by one or more energy sources. Energy sources can include, for example, mechanical, pneumatic, chemical, or electrical energy. For example, mechanical energy sources can include springs, levers, elastomers, or other mechanical mechanisms to store or release energy. One or more energy sources can be combined into a single device. Devices can further include gears, valves, or other mechanisms to convert energy into movement of one or more components of a device.

The one or more automated functions of an auto-injector may be activated via an activation mechanism. Such an activation mechanism can include one or more of a button, a lever, a needle sleeve, or other activation component. Activation may be a one-step or multi-step process. That is, a user may need to activate one or more activation mechanism in order to cause the automated function. For example, a user may depress a needle sleeve against their body in order to cause injection of a medicament. In other devices, a user may be required to depress a button and retract a needle shield in order to cause injection.

In addition, such activation may activate one or more mechanisms. For example, an activation sequence may activate at least two of needle insertion, medicament injection, and needle retraction. Some devices may also require a specific sequence of steps to cause the one or more automated functions to occur. Other devices may operate with sequence independent steps.

Some delivery devices can include one or more functions of a safety syringe, pen-injector, or auto-injector. For example, a delivery device could include a mechanical energy source configured to automatically inject a medicament (as typically found in an auto-injector) and a dose setting mechanism (as typically found in a pen-injector).

The terms "drug" or "medicament" are used herein to describe one or more pharmaceutically active compounds. As described below, a drug or medicament can include at least one small or large molecule, or combinations thereof, in various types of formulations, for the treatment of one or more diseases. Exemplary pharmaceutically active compounds may include small molecules; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more of these drugs are also contemplated.

The term "drug delivery device" shall encompass any type of device or system configured to dispense a drug into a human or animal body. Without limitation, a drug delivery device may be an drug delivery device (e.g., syringe, pen injector, auto injector, large-volume device, pump, perfusion system, or other device configured for intraocular, subcutaneous, intramuscular, or intravascular delivery), skin patch (e.g., osmotic, chemical, micro-needle), inhaler (e.g., nasal or pulmonary), implantable (e.g., coated stent, capsule), or feeding systems for the gastrointestinal tract. The presently described drugs may be particularly useful with drug delivery devices that include a needle, e.g., a small gauge needle.

The drug or medicament may be contained in a primary package or "drug container" adapted for use with a drug delivery device. The drug container may be, e.g., a cartridge, syringe, reservoir, or other vessel configured to provide a suitable chamber for storage (e.g., short- or long-term storage) of one or more pharmaceutically active compounds. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of a drug formulation (e.g., a drug and a diluent, or two different types of drugs) separately, one in each chamber. In such instances, the two chambers of the dual-chamber cartridge may be configured to allow mixing between the two or more components of the drug or medicament prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.
The drug delivery devices and drugs described herein can be used for the treatment and/or prophylaxis of many different types of disorders. Exemplary disorders include, e.g., diabetes mellitus or complications associated with diabetes mellitus such as diabetic retinopathy, thromboembolism disorders such as deep vein or pulmonary thromboembolism. Further exemplary disorders are acute coronary syndrome (ACS), angina, myocardial infarction, cancer, macular degeneration, inflammation, hay fever, atherosclerosis and/or rheumatoid arthritis.

Exemplary drugs for the treatment and/or prophylaxis of diabetes mellitus or complications associated with diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the term "derivative" refers to any substance which is sufficiently structurally similar to the original substance so as to have substantially similar functionality or activity (e.g., therapeutic effectiveness).

Exemplary insulin analogues are Gly(A21), Arg(B31), Arg(B32) human insulin (insulin glargine); Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Exemplary insulin derivatives are, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin; B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin. Exemplary GLP-1, GLP-1 analogues and GLP-1 receptor agonists are, for example: Lixisenatide / AVE0010 / ZP10 / Lyxumia, Exenatide / Exendin-4 / Byetta / Bydureon / ITCA 650 / AC-2993 (a 39 amino acid peptide which is produced by the salivary glands of the Gila monster), Liraglutide / Victoza, Semaglutide, Taspoglutide, Syncria / Albiglutide, Dulaglutide, rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.
An exemplary oligonucleotide is, for example: mipomersen / Kynamro, a cholesterol-reducing antisense therapeutic for the treatment of familial hypercholesterolemia.

Exemplary DPP4 inhibitors are Vildagliptin, Sitagliptin, Denagliptin, Saxagliptin, Berberine.

Exemplary hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Exemplary polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 / Synvisc, a sodium hyaluronate.

The term "antibody", as used herein, refers to an immunoglobulin molecule or an antigen-binding portion thereof. Examples of antigen-binding portions of immunoglobulin molecules include F(ab) and F(ab')₂ fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region.

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present disclosure include, for example, Fab fragments, F(ab')2 fragments, scFv (single-chain Fv) fragments, linear antibodies, monospecific or multispecific antibody fragments such as bispecific, trispecific, and multispecific antibodies (e.g., diabodies, triabodies, tetrabodies), minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Exemplary antibodies are anti PCSK-9 mAb (e.g., Alirocumab), anti IL-6 mAb (e.g., Sarilumab), and anti IL-4 mAb (e.g., Dupilumab).

The compounds described herein may be used in pharmaceutical formulations comprising (a) the compound(s) or pharmaceutically acceptable salts thereof, and (b) a pharmaceutically acceptable carrier. The compounds may also be used in pharmaceutical formulations that include one or more other active pharmaceutical ingredients or in pharmaceutical formulations in which the present compound or a pharmaceutically acceptable salt thereof is the only active ingredient. Accordingly, the pharmaceutical formulations of the present disclosure encompass any formulation made by admixing a compound described herein and a pharmaceutically acceptable carrier.

Pharmaceutically acceptable salts of any drug described herein are also contemplated for use in drug delivery devices. Pharmaceutically acceptable salts are for example acid addition salts and basic salts. Acid addition salts are e.g. HCI or HBr salts. Basic salts are e.g. salts having a cation selected from an alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are known to those of skill in the arts.

Pharmaceutically acceptable solvates are for example hydrates or alkanolates such as methanolates or ethanolates.

Those of skill in the art will understand that modifications (additions and/or removals) of various components of the substances, formulations, apparatuses, methods, systems and embodiments described herein may be made without departing from the full scope and spirit of the present disclosure, which encompass such modifications and any and all equivalents thereof.

List of References
- 1: drug delivery device
- 2: device housing
- 2.1, 2.2: housing part
- 2.3: mounting holes
- 6: push-rod
- 6.1: ridge
- 7: trigger
- 7.1: stem
- 10, 100, 200: servo-actuator
- 11, 111: housing
- 11.1, 111.1: grip area
- 11.2: label window, recess
- 11.3: dosage indicator window, recess
- 11.4: fixating screw
- 11.5: socket
- 11.5.1: socket collar
- 11.6: connecting flange
- 11.7: supporting collar
- 12, 112: dial grip
- 12.1: pocket
- 13, 113: button
- 13.1: protrusion
- 13.2: inner rim
- 13.2.1: recess
- 13.3: distal rim
- 14: guide sleeve
- 14.1: thread
- 14.1.1: proximal face
- 14.2: proximal rim
- 14.3: distal rim
- 15: support sleeve
- 15.1: dome
- 15.2: radial step
- 15.3: rim
- 15.4: axial slot
- 15.5: radial protrusion
- 16: pusher
- 16.1: slide
- 16.2: lead
- 16.2.1: distal face
- 16.3: spring bearing
- 16.4: receptacle
- 16.4.1: notch
- 16.5: lid
- 16.6: mechanical interface
- 17: support spring
- 18, 118: driving spring
- 19, 20: first, second ring

- 114: exterior latching sleeve
- 114.1: slot
- 114.1.1: tooth
- 114.1.2: inclined face
- 114.1.3: front face
- 114.2: stop
- 115: interior latching sleeve
- 115.1: latch arm
- 115.1.1: tooth
- 115.1.2: inclined face
- 115.1.3: front face
- 115.1.4: ridge
- 115.2: lid
- 115.3: bottom
- 116: auxiliary sleeve
- 116.1: protruding stop
- 116.2: chamfer
- 117: spring plate, support spring
- 130: pushing plate, pusher
- 130.1: rim
- 130.2: ball
- 21: controllable latch
- 216: auxiliary sleeve
- 216.1: rim
- 216.1.1: valley
- 216.1.2: hill
- 216.2: collar
- 216.3: slot
- 240: wedge sleeve, pusher
- 240.1: arm
- 240.2, 21: latch
- 240.3: beam

- D: distal end
- dX: axial displacement
- P: proximal end
- X: longitudinal axis

## Claims

1. Servo-actuator (10) for driving a push-rod (6), in particular a helically extending and returning push-rod (6) of a drug delivery device (1), the servo-actuator (10) comprising:
• a housing (11) with a connecting flange (11.6),
• the connecting flange (11.6) being mountable on a proximal section of the housing (2) of the liquid drug delivery device (1);
• a driving spring (18; 118);
• a pusher (16; 130; 240) driveable connected to the driving spring (18; 118),
• the pusher (16; 130; 240) having a mechanical interface (16.6) for engaging with the push-rod (6) of the drug delivery device (1), when mounted, such that an extension movement of the push-rod (6) results in energizing of the compression spring (18; 118) and a subsequent returning movement of the push-rod (6) is propelled by energy supplied from the compression spring (18; 118),
• the mechanical interface (16.6) further being configured for selectively interacting with a trigger (7) located at a terminal portion of the piston-rod (4) of the liquid drug delivery device (1), when mounted; and
• a controllable latch (21) for allowing a user to selectively prevent and release the pusher (16; 130; 240) from being driven by the force of the compression spring (18), when energized,
• whereby the controllable latch (21) is connected to the mechanical interface (16.6) of the pusher (16; 130; 240) such that the mechanical interface (16.6) is selected to interact with the trigger (7) when the controllable latch (21) is released.

2. Servo-actuator (10) according to claim 1, wherein the driving spring (18, 118) is configured as a linear spring or compression spring, wherein a proximal end of the driving spring (18, 118) is coupled to the housing (11) and a distal end of the driving spring (18, 118) is coupled to the pusher (16, 130, 240).

3. Servor-actuator (10) according to claim 1 or 2, wherein the driving spring (18, 118) is a pre-loaded helical spring or a preloaded spiral spring, wherein a proximal end of the driving spring (18, 118) is coupled to the housing (11) and a distal end of the driving spring (18, 118) is coupled to the pusher (16, 130, 240).

4. Servo-actuator (10) according to any of the preceding claims further comprising a dial grip (12, 112) adapted to transmit a torque to the push-rod (6).

5. Servo-actuator (10) according to any of the preceding claims, **characterised in that** the pusher (16,) is configured for coupling with the push-rod (6) of the drug delivery device (1) and for coupling with the housing (11).

6. Servo-actuator (10) according to claim 5, **characterised in that** the pusher (16) comprises a receptacle (16.4) for receiving and holding a proximal end of the push-rod (6).

7. Servo-actuator (10) according to claim 5 or 6, **characterised in that** the pusher (16) comprises a lead (16.2) for relative threaded engagement to the housing (11), in particular indirectly via the thread (14.1) of the guide sleeve (14).

8. Servo-actuator (10) according to claim 7, **characterised in that** wherein the lead (16.2) of the pusher (16) relative to the housing (11) equals a lead of the thread (14.1) which guides the push-rod (6) relative to the device housing (2).

9. Servo-actuator (10) according to any of the preceding claims further comprising a button (13) axially movable relative to the housing (11) between a proximal dialling position and a distal injection position, wherein the pusher (16, 130, 240) is engaged relative to the housing (11) when the button (13) is in its proximal dialling position and wherein the pusher (16, 130, 240) is disengaged relative to the housing (11)when the button (13) is in its distal injection position.

10. Servo-actuator (10) according to any of the preceding claims, **characterised in that** the controllable latch (21) is configured as an engagement force applied by friction of lead connections or a force fit connection between the pusher (16, 130, 240) and a support sleeve, in particular a latching sleeve (115) or a guide sleeve (14).

11. Servo-actuator (10) according to any of the preceding claims, **characterised in that** the pusher (16, 130, 240) is formed as a rotatable pusher (16) coupled with the button (13) and in threaded engagement with a non-rotatable guide sleeve (14), in particular the guide sleeve (14) is urged proximally against the pusher (16) by a support spring (17), thereby frictionally engaging the pusher (16) relative to the guide sleeve (14) such that the unloading of the driving spring (18) is frictionally inhibited when the button (13) is in its proximal dialling position and frictionally disengaging the pusher (16) relative to the guide sleeve (14) when the button (13) absorbs the spring force of the support spring (17) in its distal injection position.

12. Servo-actuator (10) according to any of the preceding claims 9 to 11, **characterised in that** the button (13) is rotationally coupled with the pusher (16) and rotationally interlaced with the guide sleeve (14), wherein the button (13) is moveable from its proximal dialling position into its distal injection position only at discrete angular positions of the pusher (16) relative to the guide sleeve (14).

13. Servo-actuator (10) according to any of the preceding claims, **characterised in that** the pusher (130, 240) is received in a radially resilient interior latching sleeve (115) that is axially slidably received in an exterior latching sleeve (114) and axially coupled with the button (13), in particular the interior latching sleeve (115) is urged proximally against the exterior latching sleeve (114) and against the button (13) by a support spring (17, 117) and further in particular the latching sleeves (114, 115) are radially interlaced such that the inner circumference of the interior latching sleeve (115) tightens in a proximal position, thereby wedging the pusher (130, 240) relative to the interior latching sleeve (115), and loosens in a distal position, thereby releasing the pusher (130, 240) relative to the interior latching sleeve (115), such that the unloading of the driving spring (18, 118) is inhibited when the button (13) is in its proximal dialling position and released when the button (13) is in its distal injection position.

14. A drug delivery device (1) with
a device housing (2) extending along a longitudinal axis (X) and receiving a cartridge (3),
a push-rod (6) with a trigger (7) protruding from the distal end of the device housing (2),
a piston rod (4) adapted to drive a piston (3.1) of the cartridge (3) with a liquid drug, and
a servo-actuator (10) according to any of the previous claims removably mounted on the device housing (2).

15. The drug delivery device (1) according to claim 14, wherein the servo-actuator (10) is configured as an add-on which is removably coupled to a proximal end (P) of the device housing (2).
